# FASCICULE DE BREVET EUROPEEN

(11) **EP 0 488 841 B1**
(45) Date de publication et mention de la délivrance du brevet: **10.01.1996**
(21) Numéro de dépôt: 91403009.3
(22) Date de dépôt: 08.11.1991
(51) Int. Cl.: A61N 1/368

(54) **Procédé de calcul de l'intervalle d'échappement à la fin duquel il faut stimuler l'oreillette d'un coeur en cas d'absence de dépolarisation**
Rechnungsverfahren des Escape-Intervalles, nach dem der Vorhof, im Falle von nicht-Depolarisation stimuliert werden muss
Method for calculating the escape interval after which the atria have to be paced in case of absence of depolarisation

(30) Priorité: 30.11.1990 FR 9015012
(43) Date de publication de la demande: 03.06.1992
(73) Titulaire: ELA MEDICAL (Société anonyme), F-92541 Montrouge Cédex (FR)
(72) Inventeur: Girodo, Sylvie, F-92120 Montrouge (FR); Malherbe, Odile, F-94230 Cachan (FR)
(74) Mandataire: Laget, Jean-Loup

(56) Documents cités:
- US-A- 4 313 442
- US-A- 4 515 161
- US-A- 4 543 963
- US-A- 4 569 350

## Description

La présente invention concerne d'une manière générale la stimulation du coeur d'un patient à l'aide d'un stimulateur cardiaque double chambre.

De tels appareils, connus et utilisés de longue date, permettent de réguler l'activité cardiaque du ventricule en utilisant l'information recueillie au niveau de l'oreillette du coeur du patient en cas de défaillance du noeud auriculo-ventriculaire, qui induit alors un blocage, au niveau des ventricules, de la réponse à une dépolarisation des oreillettes.

Un tel stimulateur détecte la dépolarisation d'une oreillette par ce que les cardiologues nomment l'onde P et stimule le ventricule, si celui-ci ne s'est pas dépolarisé physiologiquement après un délai auriculo-ventriculaire donné.

Le stimulateur est également capable d'éviter de stimuler le ventricule en réponse à des ondes P trop rapprochées afin de ne pas fatiguer le coeur inutilement.

Un stimulateur double chambre est de plus apte, à stimuler l'oreillette lorsqu'il n'y a plus de détection auriculaire.

De manière connue en soi, lorsqu'il n'y a plus de détection auriculaire, le stimulateur stimule l'oreillette, puis, après un délai auriculo-ventriculaire, il stimule le ventricule.

Actuellement, deux méthodes sont mises en oeuvre lors d'une telle absence de détection auriculaire.

Dans certains stimulateurs, on impose immédiatement au coeur une stimulation de fréquence égale à la fréquence de base, c'est-à-dire la fréquence minimale que peut supporter le coeur du patient.

Dans d'autres stimulateurs, on a préféré poursuivre la stimulation à la fréquence mémorisée avant l'absence de détection auriculaire puis réduire peu à peu cette fréquence jusqu'à la fréquence de base.

Le document US 4 313 442 décrit un stimulateur dans lequel le rythme de stimulation décroît jusqu'à la fréquence de base.

La fréquence de base est définie par la valeur la plus grande que peut prendre un intervalle d'échappement, correspondant au temps à la fin duquel il faut obligatoirement stimuler l'oreillette si la dépolarisation de celle-ci n'est pas détectée.

Cet intervalle d'échappement est régulièrement recalculé si bien que sa valeur a tendance à se rapprocher de plus en plus de la valeur séparant deux ondes P successives.

L'inconvénient est que tout évènement auriculaire quel qu'il soit est pris en compte lors du calcul de cet intervalle d'échappement auriculaire (IEA). Lors de rythmes rapides auriculaires fréquents, on aboutit à un rythme d'échappement de plus en plus rapide.

Actuellement, il est connu d'effectuer ce calcul en mesurant le délai entre deux ondes P tous les huit cycles, ou après chaque cycle, le cycle étant défini par l'ensemble d'une dépolarisation auriculaire et de la dépolarisation auriculaire suivante.

Une telle méthode conduit à prendre le risque assez grand pour certains patients de faire la mesure lors d'une extrasystole auriculaire, c'est-à-dire d'une dépolarisation auriculaire spontanée prématurée. Si tel est le cas, le délai entre les deux ondes P successives est anormalement court ce qui entraîne le calcul d'un intervalle d'échappement auriculaire très court et une stimulation accélérée de l'oreillette tendant à stimuler le coeur à une fréquence inutilement élevée en cas d'absence de détection auriculaire.

La présente invention tend à proposer un nouveau procédé de calcul qui permette de s'affranchir d'un tel risque.

L'invention a pour objet un procédé de calcul de l'intervalle d'échappement auriculaire à la fin duquel il faut stimuler l'oreillette d'un coeur en cas d'absence de dépolarisation, caractérisé en ce que l'on calcule la valeur moyenne des délais entre deux ondes P mesurés sur un nombre de cycles cardiaques ne présentant pas d'extrasystole compris entre 2 et 12 et de préférence égal à 8, et on prend cette valeur moyenne pour valeur de l'intervalle d'échappement auriculaire, l'absence d'extrasystole étant déduite d'un examen du rythme cardiaque.

Selon d'autres caractéristiques de l'invention :
- on déclenche après toute détection auriculaire une période réfractaire auriculaire post auriculaire (PRAPA) d'une durée égale à une fraction du temps séparant deux ondes P sans dépasser un délai programmable au cours de laquelle toute onde P détectée est interprétée comme une extrasystole,
- ladite fraction du temps séparant deux ondes P est de 75% et le délai programmable est compris entre 300 et 700ms et de préférence égal à 560ms,
- lorsqu'une onde P est détectée au cours de la PRAPA, on ne déclenche pas de délai auriculo-ventriculaire et on déclenche un nouvel intervalle d'échappement auriculaire, pour éviter une stimulation trop rapide du ventricule,
- on observe si une onde P a été détectée en PRAPA afin de déterminer si le cycle cardiaque comporte une extrasystole auriculaire.

D'autres caractéristiques ressortent de la description qui suit faite avec référence au dessin annexé sur lequel on peut voir :
Figure 1 : un schéma représentatif de la mise en oeuvre du procédé de calcul de l'intervalle d'échappement auriculaire;
Figure 2 : un schéma représentatif de la mise en oeuvre du procédé selon l'invention dans le cas d'une extrasystole auriculaire ;
Figure 3 : un diagramme explicatif du fonctionnement d'un stimulateur cardiaque mettant en oeuvre le procédé selon l'invention.

Le procédé selon l'invention calcule une valeur moyenne du délai entre deux ondes P successives à partir de mesures prises sur un nombre de cycles cardiaques compris entre 2 et 12 et de préférence égal à 8, ne comportant pas d'extrasystole.

Pour la mise en oeuvre de ce procédé, il est donc nécessaire d'utiliser un stimulateur cardiaque capable de déterminer si une accélération du rythme cardiaque est physiologique ou pathologique.

L'accélération sera physiologique lorsque le patient sera en phase d'effort plus ou moins prolongé, tandis qu'elle sera pathologique en présence d'extrasystoles auriculaires.

A cet effet, on a défini une période réfractaire absolue très courte, après une stimulation du ventricule, durant laquelle on ne détecte aucun évènement dans l'oreillette. Celle-ci est limitée au temps de repos nécessaire aux amplificateurs après la stimulation.

Cette période réfractaire absolue courte permet d'améliorer le temps d'écoute au niveau de l'oreillette et d'examiner toute accélération du rythme cardiaque afin de déterminer si l'on est dans le cas d'un effort physique ou d'une extrasystole.

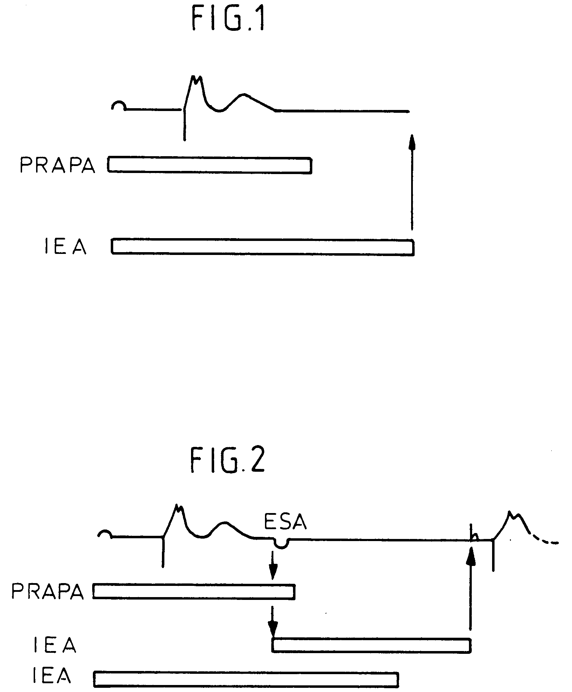

Ainsi, on définit une période réfractaire auriculaire post auriculaire (PRAPA) dont la valeur est une fraction du temps moyen séparant deux ondes P, par exemple égale à 75% du temps séparant les deux ondes P du cycle précédent sans dépasser un délai programmable qui peut être compris entre 300 et 700 ms et de préférence égal à 560ms (Figure 1).

On considère en effet qu'une accélération physiologique n'est jamais capable de générer une onde P avec une accélération supérieure à 25%.

Le stimulateur, refuse alors de prendre en compte une onde P se produisant avant la fin de cette PRAPA déterminée mise en oeuvre après la détection d'une première onde P. Lorsqu'une telle onde P intervient dans ce délai (Figure 2), elle est interprétée comme une extrasystole isolée (ESA). Mais le stimulateur déclenche un nouvel intervalle d'échappement auriculaire. A la fin de ce nouvel IEA, on stimule l'oreillette, et après un délai auriculo-ventriculaire, on stimule le ventricule.

Il est alors aisé, en observant si une onde P a été ou non détectée en PRAPA, de déterminer si une extrasystole a eu lieu ou non durant le cycle, et ainsi, de ne mesurer les délais qu'entre les ondes P compétentes successives. On nomme usuellement onde P compétente une onde P qui peut être effectivement suivie d'une stimulation du ventricule.

Suivant le procédé selon la présente invention, le délai entre deux ondes P est mesuré uniquement lorsque les ondes P sont compétentes et ceci, à chaque fois, pour huit cycles de préférence.

On calcule alors la moyenne de ces huit valeurs que l'on prend comme valeur du délai entre deux ondes P.

Cette valeur est alors comparée à celle de l'intervalle d'échappement.

Lorsque l'intervalle d'échappement est beaucoup plus grand que le délai entre deux ondes P, il est réduit afin de stimuler le coeur suivant un rythme tenant compte de l'accélération éventuelle de l'activité du patient.

Lorsque l'oreillette a été stimulée pendant un certain nombre de cycles, 8 par exemple, l'intervalle d'échappement auriculaire est prolongé d'une valeur comprise entre 16 et 94 ms, et de préférence égale à 47 ms, jusqu'à atteindre l'intervalle correspondant à la fréquence de base programmée.

Un tel procédé permet ainsi une meilleure adéquation entre l'activité cardiaque et l'activité du patient lorsque l'on détecte une absence de dépolarisation auriculaire.

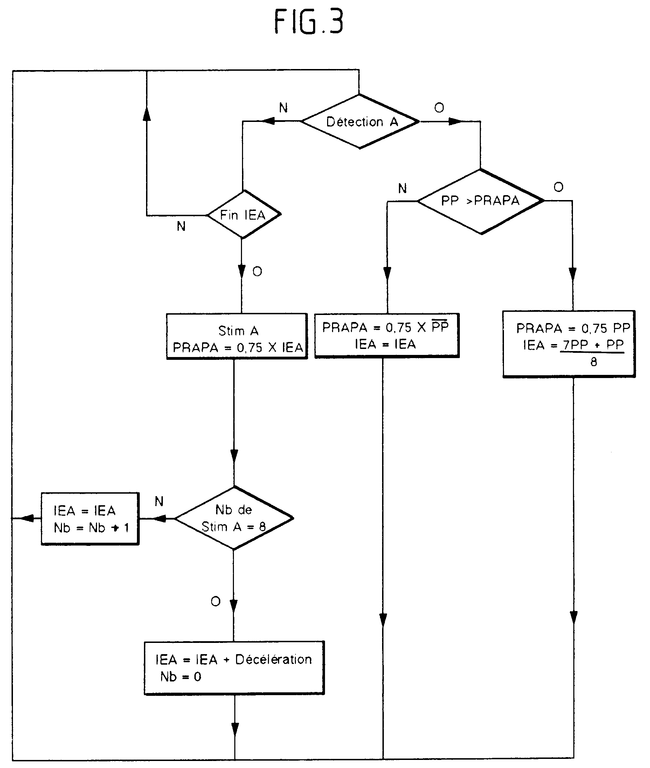

Le diagramme de la Fig. 3 récapitule ces différentes opérations, avec les abréviations suivantes :
- Détection A: : détection auriculaire ;
- Fin IEA: : fin de l'intervalle d'échappement auriculaire;
- Stim A: : stimulation auriculaire ;
- Nb de Stim A: : nombre de stimulations auriculaires sur des cycles consécutifs ;
- Décélération: : prolongation de l'IEA de 47ms ;
- Nb = O: : remise à zéro du compteur de stimulations;
- PP :: : intervalle entre deux ondes P ;
- PP: : moyenne des intervalles PP sur 8 cycles ;
- O: : oui ;
- N: : non.

## Revendications

1. Procédé de calcul de l'intervalle d'échappement auriculaire, à la fin duquel il faut stimuler l'oreillette d'un coeur en cas d'absence de dépolarisation, caractérisé en ce que l'on calcule la valeur moyenne des délais entre deux ondes P mesurés sur un nombre de cycles cardiaques ne présentant pas d'extrasystole compris entre 2 et 12 et de préférence égal à 8, et on prend cette valeur moyenne pour valeur de l'intervalle d'échappement auriculaire, l'absence d'extrasystole étant déduite d'un examen du rythme cardiaque.

2. Procédé selon la revendication 1, caractérisé en ce que l'on déclenche après toute détection auriculaire une période réfractaire auriculaire post auriculaire (PRAPA) d'une durée égale à une fraction du temps séparant deux ondes P sans dépasser un délai programmable au cours de laquelle toute onde P détectée est interprétée comme une extrasystole.

3. Procédé selon la revendication 2, caractérisé en ce que ladite fraction du temps séparant deux ondes P est de 75%, et le délai programmable est compris entre 300 et 700 ms et de préférence égal à 560ms.

4. Procédé selon la revendication 2, caractérisé en ce que lorsqu'une onde P est détectée au cours de la PRAPA, on ne déclenche pas de délai auriculo-ventriculaire et on déclenche un nouvel intervalle d'échappement auriculaire.

5. Procédé selon la revendication 3, caractérisé en ce que l'on observe si une onde P a été détectée en PRAPA afin de déterminer si le cycle cardiaque comporte une extrasystole auriculaire.

## Claims

1. A method for calculating the atrial escape interval at the end of which it is necessary to stimulate the atrium of a heart in the event of absence of depolarisation, characterised in that the mean value of the delays between two P waves measured over a number of cardiac cycles which do not have an extrasystole of between 2 and 12 and preferably equal to 8 is calculated, and this mean value is taken as the value of the atrial escape interval, the absence of extrasystole being deduced from examination of the acceleration of the heart rate.

2. A method according to Claim 1, characterised in that after any atrial detection a post-atrial atrial refractory period (PRAPA), of a duration equal to a fraction of the time between two P waves without exceeding a programmable delay is triggered, during which any P wave detected is interpreted as an extrasystole.

3. A method according to Claim 2, characterised in that said fraction of the time between two P waves is 75%, and the programmable delay is between 300 and 700 ms, and preferably equal to 560 ms.

4. A method according to Claim 2, characterised in that when a P wave is detected during the PRAPA no atrioventricular delay is triggered, and a new atrial escape interval is triggered.

5. A method according to Claim 3, characterised in that one observes whether a P wave has been detected in PRAPA in order to determine if the cardiac cycle comprises an atrial extrasystole.

## Patentansprüche

1. Berechnungsverfahren des aurikulären Ausströmintervalls, an dessen Ende das Herzohr eines Herzens in Fall des Fehlens der Depolarisation stimuliert werden muß,
**dadurch gekennzeichnet, daß**
der Mittelwert der zwischen zwei "P"-Zacken auftretenden Verzögerungen berechnet wird, welche über einer Anzahl von zwischen 2 und 12, vorzugsweise 8, keine Extrasystole aufweisenden Herzzyklen gemessen werden, und dieser Mittelwert als Wert des aurikulären Ausströmintervalls festgelegt wird, wobei das Fehlen der Extrasystole aus einer Untersuchung der Beschleunigung des Herzrythmus abgeleitet wird.

2. Verfahren gemäß Patentanspruch 1,
**dadurch gekennzeichnet, daß**
nach jeder aurikulären Erfassung eine aurikulär-postaurikuläre Refraktärphase (PRAPA) ausgelöst wird, deren Dauer gleich einem Bruchteil der Zeit ist, die zwei "P"-Zacken trennt, ohne eine programmierbare Verzögerung zu überschreiten, in deren Verlauf jede erfaßte "P"-Zacke als eine Extrasystole interpretiert wird.

3. Verfahren gemäß Patentanspruch 2,
**dadurch gekennzeichnet, daß**
der Bruchteil der zwei "P"-Zacken trennenden Zeit gleich 75% ist, und die programmierbare Verzögerung zwischen 300 und 700 ms liegt und vorzugsweise 560 ms beträgt.

4. Verfahren gemäß Patentanspruch 2,
**dadurch gekennzeichnet, daß**
wenn eine "P"-Zacke im Verlauf der PRAPA erfaßt wird, keine aurikulär-ventrikuläre Verzögerung ausgelöst wird und ein neues aurikuläres Ausströmintervall ausgelöst wird.

5. Verfahren gemäß Patentanspruch 3,
**dadurch gekennzeichnet, daß**
beobachtet wird, ob eine "P"-Zacke während der PRAPA erfaßt wurde, um zu bestimmen, ob der Herzzyklus eine aurikuläre Extrasystole aufweist.
